Europäisches Patentamt

(19) European Patent Office

Office européen des brevets

(11) Numéro de publication: **0 211 918**
**B1**

(12) **FASCICULE DE BREVET EUROPEEN**

(45) Date de publication du fascicule du brevet:
**26.07.89**

(51) Int. Cl.⁴: **A 61 B 17/34**

(21) Numéro de dépôt: **86901410.0**

(22) Date de dépôt: **20.02.86**

(86) Numéro de dépôt international:
**PCT/FR 86/00052**

(87) Numéro de publication internationale:
**WO 86/04805 (28.08.86** Gazette 86/19)

(54) **DISPOSITIF DE PONCTION DE MOELLE.**

(30) Priorité: **20.02.85 FR 8502452**

(43) Date de publication de la demande:
**04.03.87 Bulletin 87/10**

(45) Mention de la délivrance du brevet:
**26.07.89 Bulletin 89/30**

(84) Etats contractants désignés:
**AT BE CH DE FR GB IT LI LU NL SE**

(56) Documents cités:
**EP-A- 0 115 388**
**DE-A- 3 108 766**
**FR-A- 1 190 905**
**FR-A- 2 138 152**
**FR-A- 2 393 583**
**US-A- 4 239 040**

(73) Titulaire: **BIOLOGIE & INDUSTRIE S.A.R.L., 34, rue de Constantinople, F-75008 Paris (FR)**

(72) Inventeur: **BROSSEL, Rémy, 31, rue de Moscou, F-75008 Paris (FR)**

(74) Mandataire: **Gutmann, Ernest et al, S.C. Ernest Gutmann - Yves Plasseraud 67, boulevard Haussmann, F-75008 Paris (FR)**

## Description

La moelle sanguine, ou hématopoïétique, est un organe semi-liquide, d'environ 5 litres, et contenue dans les os. La moelle fabrique les globules qui vont ensuite circuler dans le sang, après avoir pénétré dans le «secteur circulant», c'est-à-dire le volume disponible dans les artères et les veines.

Depuis plusieurs décennies, on pratique des «ponctions de moelle» ou «ponctions médullaires» pour prélever un échantillon de moelle hématopoïétique nécessaire à la réalisation des examens biologiques. Ces examens sont notamment demandés par les médecins hématologues pour des anomalies graves des globules du sang, au premier rang desquelles les leucémies, qui sont des maladies médullaires. Les matériels utilisés sont en général spécifiquement conçus pour ce type de ponctions, essentiellement en raison, d'une part, de la nature semi-liquide de l'organe prélevé, la moelle, et, d'autre part, de la nécessité pour atteindre la moelle, de traverser un autre organe dur, l'os. Seule la moelle présente ces deux particularités.

Ces matériels comprennent en général des trocarts aptes à traverser l'os du sternum, d'où le nom, également très fréquent de ponction sternale. En France, on choisit en règle un trocart de Mallarmé, enfoncé manuellement à travers l'os. Ce trocart est de diamètre variable, de 8 à 20 centièmes de millimètre de diamètre intérieur, et de longeur suffisants pour traverser l'épaisseur (de l'ordre du centimètre) d'os dur — ou «compact» — qui entoure la moelle. On dit que l'on traverse la corticale pour atteindre la médullaire. L'extrémité biseautée du trocart une fois en place dans l'espace médullaire, il faut exercer une légère dépression, à l'aide d'une seringue pour aspirer de 0,5 à 2 ml de moelle ; en effet elle est assez visqueuse, et l'espace médullaire est cloisonné en multiples logettes par de l'os, peu résistant, dit lamellaire.

La moelle est recueillie dans la seringue, sur anticoagulant pour éviter la formation d'un caillot. On retire ensuite l'ensemble seringue-trocart. Le trocart seul sera nettoyé, stérilisé, réutilisé. La seringue sert à déposer une goutte de moelle sur une lame. Cette goutte est étalée, séchée, puis envoyée au laboratoire qui va la colorer, puis l'examiner au microscope.

Un dispositif selon le préambule de la revendication 1 est connu du document FR-A-2 138 152.

Il résulte de ce qui précède que ces prélèvements de moelle constituent un acte traumatisant pour le patient. A cet inconvénient majeur, inévitable jusqu'à ce jour, s'ajoute le caractère souvent difficilement reproductible des prélèvements.

L'invention a pour but de remédier au moins en partie à ces difficultés, notamment de fournir des matériels de prélèvement de moelle qui soient rendus à la fois moins traumatisants pour les malades, plus fiables pour les médecins ou manipulateurs, et qui de surcroît soient d'un coût à ce point réduit qu'ils soient «jetables», de sorte que soient éliminés tous les problèmes de stérilisations qui doivent être répétées à ce jour entre chacune des utilisations successives de ces matériels.

Le dispositif selon l'invention comprend une aiguille de prélèvement traversant un piston et solidarisée avec celui-ci, ce piston étant susceptible de se déplacer à l'interieur d'un corps de piston ou tube entre une première position et une seconde position, caractérisé

— en ce que le piston est maintenu dans sa première position contre l'action de moyens exerçant sur lui une force tendant à l'entraîner vers la seconde position, par des moyens de retenue susceptibles d'être commandés de l'extérieur pour libérer le mouvement dudit piston sous l'action des premiers moyens susmentionnés,

— en ce que dans ladite première position l'aiguille est entièrement escamotée à l'intérieur de la partie antérieure dudit corps de piston, et en ce que la course du piston est telle que, lorsque la partie antérieure du dispositif est placée et maintenue par le manipulateur, directement ou par le truchement d'un système externe de support co-agissant avec ce dispositif contre le corps du malade ou à une distance déterminée du corps du malade, au niveau de l'os qui doit être traversé par l'aiguille, l'extrémité de l'aiguille soit apte à sortir du corps de piston au niveau de son extrémité antérieure, notamment à travers un opercule ou analogue, à traverser l'épaisseur de l'os et à atteindre la région de la moelle où devra s'effectuer le prélèvement, lorsque le piston aura été soustrait à l'action desdits moyens de retenue par l'intermédiaire desdits moyens commandés de l'extérieur et

— en ce que la partie postérieure du corps de piston, de l'autre côté du piston, définit une chambre fermée pour la réception de la moelle prélevée et aspirée dans cette chambre, sous l'effet de la dépression qui est alors formée consécutivement au déplacement du piston de sa première position vers sa deuxième position.

Il va sans dire que les moyens qui tendent à entraîner le piston vers ladite seconde position doivent effectivement exercer une force suffisante à assurer le transpercement du sternum ou d'un autre os donnant accès à la moelle et l'accès de l'aiguille dans l'espace médullaire. Ces moyens sont de préférence du type élastique. Avantageusement ils sont constitués par un ressort travaillant en traction.

Dans un mode de réalisation préféré du dispositif selon l'invention, celui-ci comporte encore un ou plusieurs compartiments distincts de la chambre de réception, néanmoins susceptibles d'être mis en communication avec celle-ci. Ce ou ces compartiments peuvent contenir des réactifs divers, liquides ou solides, par exemple des milieux de conservation. Dans une forme préférée du dispositif selon l'invention, la séparation entre la chambre de réception et un tel compartiment est réalisée par l'intermédiaire d'un mince disque ou pellicule, par exemple en aluminium ou matière plastique, fracturable sous l'effet de la dépression consécutive au déplacement du piston de la première à la seconde position. Ce compartiment distinct contient par exemple des réactifs qui peuvent ainsi être immédiatement mélangés avec la quantité de moelle qui reflue dans la chambre susdite.

Les moyens élastiques tendant à entraîner le piston de la première vers la seconde position sont avantageusement constitués par un ressort travaillant en traction. Les moyens susceptibles d'être commandés de l'extérieur pour libérer le mouvement dudit piston sous l'action dudit ressort sont par exemple constitués par des doigts ou ergots contre lesquels bute le piston, ces doigts ou ergots étant escamotables à l'intérieur de l'épaisseur des parois du corps de piston ou contre celles-ci, sous l'effet de la commande extérieure. Celle-ci peut être constituée de toute façon appropriée, par exemple par un dispositif d'armement mécanique extérieur, par exemple un système de déclenchement-enclanchement, associé ou susceptible d'être associé avec le corps de piston. Ce système de déclenchement est par exemple monté dans un système pourvu d'un logement le rendant apte à recevoir l'ensemble du corps de piston. Ce système externe comporte avantageusement, en sus de ce logement, une poignée convenablement placée, vis-à-vis du dispositif selon l'invention, de façon à permettre l'application de sa partie antérieure ou de rebords antérieurs correspondants dudit système, contre la région appropriée du corps du malade, de manière à assurer un contact correct, adhérent à la peau, et enfin une gâchette de déclenchement, associée auxdits ergots escamotables par l'intermédiaire d'organes cinématiques appropriés. Cette poignée ergonomique doit faciliter une bonne prise, libérer un doigt pour le déclenchement, et assurer l'amortissement mécanique, avec la main, du choc retour du piston.

Avantageusement le dispositif selon l'invention forme une «cartouche» logeable dans le susdit système de support, ce système de support étant en outre pourvu de moyens permettant la mise en liaison mécanique ou autre entre les susdits moyens de retenue et l'organe de commande, par exemple la gâchette susdite.

Les dispositifs de l'invention permettent la mécanisation et/ou l'automation du recueil de moelle dans l'espace médullaire. La constance chez tous les individus de la profondeur d'os à traverser pour atteindre la moelle permet l'obtention ou le recueil d'échantillons de volumes également constants et susceptibles d'être considérés comme représentatifs de la moelle hématopoiétique, dès lors que les caractéristiques dimensionnelles et de force des moyens élastiques ou autre mis en oeuvre sont également constants d'une cartouche à l'autre.

On obtient ainsi des dispositifs permettant de réduire à un minimum le caractère traumatisant pour le malade de l'acte médical que requiert le prélèvement de moelle. Le dispositif selon l'invention satisfait à deux des buts que s'assignait l'invention, en particulier rendre plus rapide la ponction sternale dans ces deux composantes (traversée de l'os et aspiration de la moelle) et améliorer les conditions de l'acte pour le praticien lui-même et le caractère reproductible du prélèvement, le contact avec le patient étant médianisé par l'intermédiaire de l'extrémité du dispositif selon l'invention (ou par les parties de l'organe associées avec ce dispositif et comportant les moyens de commande) appliquée contre le sternum du patient. L'acte commandé par le practicien est alors essentiellement limité au déclenchement du mouvement du piston et de l'aiguille de prélèvement qui lui est associée. Enfin la mécanisation possible de l'acte permet la réalisation de prélèvements de qualités reproductibles.

Un avantage supplémentaire du dispositif selon l'invention réside dans son faible coût. En effet, les éléments essentiels de ce dispositif, que constituent l'aiguille de prélèvement, le piston qui lui est associé et le corps de piston, peuvent être réalisés dans des matériaux bon marché. Dans sa forme la plus avantageuse, les dispositifs selon l'invention sont constitués de cartouches destinées à ne servir qu'une seule fois. En d'autres termes, elles peuvent être du type «jetable» après usage.

Le corps du dispositif, y inclus la susdite chambre, peut également être utilisé comme moyen de préservation pendant le temps nécessaire au transport de l'échantillon vers le laboratoire. Avantageusement, le corps est réalisé en deux parties séparables l'une de l'autre, par exemple par vissage mutuel, avec pour conséquence la possibilité d'accéder au contenu de la chambre au moment de la réalisation des analyses.

Des caractéristiques supplémentaires de l'invention apparaîtront encore au cours de la description qui suit de modes de réalisation préférés du dispositif selon l'invention, à l'aide du dessin dans lequel:

— la fig. 1 est une section à travers une réalisation du dispositif selon l'invention, du type cartouche, dans laquelle le piston est maintenu dans sa «première position»,

— la fig. 2 représente une cartouche du même type associé avec un organe support ou récepteur de la cartouche, cet organe support étant pourvu de moyens permettant la commande du déclenchement du mouvement du piston, celui-ci étant néanmoins encore représenté dans sa «première position»,

— la fig. 3 est le schéma du même système, après déclenchement du mouvement du piston, celui-ci se trouvant alors dans sa «seconde position».

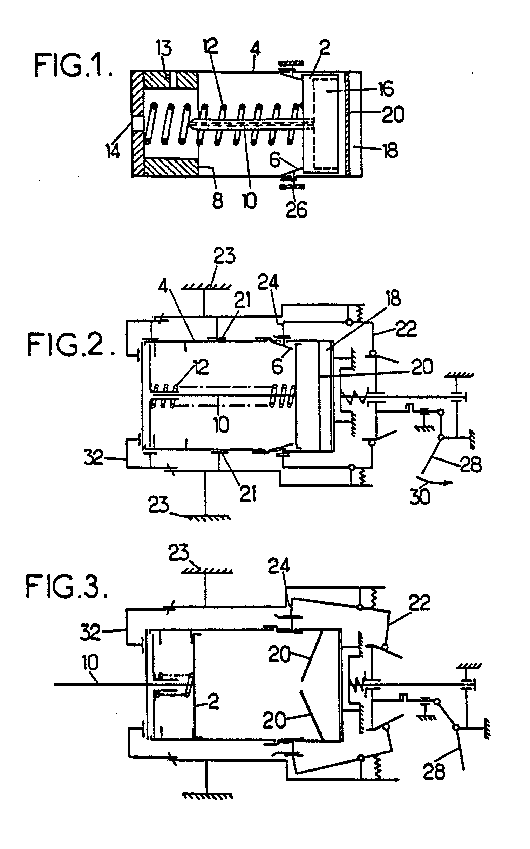

La fig. 1 montre les éléments essentiels du dispositif de prélèvement selon l'invention. Il comporte un piston 2 susceptible de se déplacer à l'interieur d'un corps 4, généralement tubulaire, entre les susdites première et seconde positions, respectivement déterminées par des doigts ou ergots internes 6 et par une butée 8. Le piston est maintenu dans sa première position par l'intermédiaire desdits doigts et butées, lesquels dans la fig. 1 sont représentés comme projetés vers l'intérieur à partir des parois internes du corps. L'aiguille 10, y compris sa pointe convenablement biseautée, est alors en position de repos, à l'intérieur du corps.

Par escamotage de ces doigts ou ergots à l'intérieur de la paroi du corps (ou par rétraction de ces doigts vers la surface intérieure de ce corps), le piston et l'aiguille creuse 10 qui lui est associée sont entraînés vers la seconde position par l'intermédiaire d'un ressort 12 travaillant en traction, le mouvement du piston vers sa seconde position étant interrompu lorsqu'il vient s'appliquer sur les susdites butées 8. De préférence, un orifice latéral 13 dans le corps du piston, en aval de la «deuxième position» du piston dans le sens de son déplacement permet l'échappement de l'air qui, autrement, s'opposerait partiellement à la force de lancement du piston.

La course du piston est calculée pour que l'aiguille qui l'accompagne dans son mouvement soit apte à traverser le fond correspondant du corps du piston (initialement fermé par un opercule 14 que traverse l'aiguille), à traverser l'os et à venir se loger dans la région de la moelle où doit s'effectuer le prélèvement, lorsque ledit dispositif aura été maintenu appliqué contre le sternum du patient et maintenu dans cette position pendant que s'effectue le déplacement du piston et de l'aiguille sous l'effet du ressort de traction 12. Il va de soi que la force de ce ressort doit être calculée, pour que l'aiguille soit effectivement rendue apte à traverser l'os du sternum.

Comme également visible sur la fig. 1, le piston est avantageusement creux et celui-ci définit dans la partie opposée du corps une chambre 14 pour la réception de la moelle prélevée et aspirée dans cette chambre, sous l'effet de la dépression formée dans celle-ci, suite au déplacement du piston de sa première position vers sa deuxième position.

La fig. 1 fait également apparaître l'existence d'un compartiment distinct 18, susceptible d'être mis en communication avec la chambre 16. Dans le mode de réalisation représenté, la chambre 16 et le compartiment 18 sont séparés par une pellicule 20 fracturable, notamment sous l'effet de la dépression produite quand le piston est entraîné de la première à la seconde position sous l'action du ressort 12.

On conçoit que le dispositif de la fig. 1, qui se présente sous forme d'une cartouche, puisse être intégré dans un système de support schématisé dans les fig. 2 et 3 et comportant, notamment, des éléments de cloisons 21 définissant un logement pour la cartouche et des organes de commande susceptibles d'être mis en communication avec les doigts ou organes bloquant le piston dans sa première position, et des organes de supports appartenant à la poignée ergonomique (non représentée à l'exception des parties schématisées en 23). Cette mise en place comprendra notamment la mise en communication entre des organes de commande schématisés en 22 avec les ergots ou doigts 6 comme schématiquement représentés en 24. On remarquera d'ailleurs à cet effet, qu'avant cette mise en communication, les ergots ou doigts 6 des cartouches pouvaient par exemple être maintenus en place par l'intermédiaire d'une rondelle 26 de matière élastique, notamment en caoutchouc, ou tout autre moyen analogue, cette rondelle étant enlevée ou éjectée à l'occasion de l'introduction desdites cartouches dans le logement du dispositif support.

La commande de l'effacement vers l'intérieur des doigts ou ergots 6 est, par exemple, commandée par l'intermédiaire d'un mécanisme d'armement à gâchette 28, actionnable par exemple dans la direction indiquée par la flèche 30.

La fig. 3 fait apparaître les conditions dans lesquelles le mécanisme d'armement a été relâché par armement de la gâchette 28. L'effacement des ergots a eu pour conséquence le mouvement rapide du piston et de l'aiguille solidarisée avec lui vers la susdite seconde position. Il est naturellement clair que, dans la construction envisagée, la longueur du piston est telle que la longueur de la partie de l'aiguille faisant saillie vis-à-vis de la surface 32 d'application de l'ensemble du système sur le sternum du patient corresponde à la distance de pénétration nécessaire de l'aiguille pour que son extrémité se trouve en définitive logée au milieu de la moelle à prélever.

La fig. 3 représente également la pellicule de séparation 20, qui antérieurement séparait la chambre 16 et le compartiment 18, dans un état de fracturation, conséquence de la dépression qui a accompagné le mouvement brutal du piston de sa première position à sa seconde position, sous l'action du ressort 10. On conçoit bien que le dispositif selon l'invention permet en quelque sorte la réalisation d'un mélange in situ de la moelle aspirée dans la chambre 16 avec un liquide initialement présent dans le compartiment 18. Il va naturellement de soi que tout autre mode d'utilisation du dispositif peut être envisagé, impliquant par exemple le mélange de la moelle séparée avec des produits secs déposés sur la membrane 20 ou même (en l'absence éventuelle de membrane) sur les parois ou le fond de la chambre à l'opposé de l'extrémité au travers de laquelle l'aiguille est appelée à être projetée vers l'extérieur.

Il va de soi que plus généralement la longueur de l'aiguille, le volume du corps du piston, les volumes intérieurs des chambres sont calculés de façon à permettre le prélèvement de la quantité de moelle désirée.

Par exemple des dimensions adéquates des parties essentielles du dispositif ou de la cartouche sont les suivantes:

— course du piston: longueur en saillie nécessaire à l'enfoncement de l'aiguille dans le sternum sur une longueur     15 mm

— section interne de la cartouche (valeurs usuelles)     2 à 4 cm$^2$

— diamètre interne de l'aiguille creuse (valeurs usuelles)     0,05 à 0,30 mm.

Il va de soi que tout autre système de commande que celui qui a été décrit peut être utilisé. Le système à ergots peut être remplacé par un dispositif de commande électromécanique ou électromagnétique. L'énergie nécessaire au déplacement du piston de sa première à sa seconde position peut être fournie par d'autres moyens, par exemple des moyens pneumatiques, ou par le maintien d'une pression d'air à l'intérieur de l'espace destiné à être parcouru par le piston contre les moyens exerçant une force en sens opposé, le déclenchement du mécanisme mettant en jeu une mise à l'air de cet espace, etc..

Le dispositif de support peut être réalisé de façon quelconque. Avantageusement, il comprend une poignée (non représentée) ergonomique, permettant une bonne prise de l'appareillage, l'application efficace de la zone d'extrémité 32 sur le sternum du malade, l'amortissement mécanique avec la main du choc en retour du piston, lorsque celui-ci se meut rapidement de la première à la seconde position dès lors que les doigts ou ergots avaient été libérés sous l'effet de la commande par l'intermédiaire de la gâchette.

Ce système est alors prêt à fonctionner.

Après déclenchement, le piston parcourt un trajet suffisant pour que l'aiguille perce l'opercule 14, traverse un centimètre d'os compact chez l'adulte et se retrouve à environ 0,5 cm de profondeur dans la moelle. La dépression créée par le mouvement du piston produit le reflux à travers l'aiguille de 0,5 à 1 cm³ de moelle et entraîne le décollement ou la fracture de la membrane ou du disque métallique 20.

Les applications de ce système sont multiples. Par exemple on réalise ainsi le mélange de la moelle refoulée dans la chambre 16 avec un milieu liquidien de culture, éventuellement hépariné ou anticoagulé et, par exemple encore, avec un anticorps déterminé initialement retenu sur la membrane, cet anticorps passant alors en solution dans le milieu. L'anticorps est par exemple destiné à la détection d'antigènes membranaires particuliers ou de cellules étrangères à la moelle.

L'échantillon peut être agité pour obtenir une bonne homogénisation avant d'être envoyé au laboratoire, la conservation pouvant être, en milieu de culture, de quelques heures à la température ordinaire. Ceci assure la conservation des antigènes de membranes nécessaires à la détection des cellules étrangères à la moelle, ou à l'étude de populations de cellules souches ou différenciées. A titre d'autres usages indiqués à titre d'exemples, et auxquels le dispositif selon l'invention peut donner lieu, on mentionnera:

— le transport direct ou différé au laboratoire d'un échantillon prélevé avec un simple receuil sur anticoagulant,

— la conservation de la moelle prélevée dans un milieu adéquat,

— le traitement à l'intérieur même de l'appareil du contenu prélevé, puis la réinjection du total (cas dans lequel le système pourra être modifié pour autoriser une commande du retour du piston à partir de sa «deuxième position» vers sa «première position»),

— le marquage de certaines catégories de cellules médullaires par un moyen quelconque, enzymatique, immunologique, radioactif ou autre,

— la recherche de cellules anormales ou étrangères à la moelle,

— le transfert à un appareil de mesure comme un cytomètre en flux liquide, ou à une centrifugeuse, ou à un système de tri, voire de fixation et d'incorporation à l'intérieur de l'appareil.

Comme il va de soi et comme il résulte d'ailleurs déjà de ce qui précède, l'invention ne se limite nullement à ceux de ses modes d'application et de réalisation qui ont été plus spécialement envisagés; elle en embrasse au contraire toutes les variantes.

**Revendications**

1. Dispositif de ponction de moelle comprenant une aiguille (10) solidarisée avec un piston (2), ce piston étant susceptible de se déplacer entre une première position et une seconde position à l'intérieur d'un corps de piston ou tube (4) pourvu d'une partie antérieure,

— ce piston (2) étant maintenu dans sa première position contre l'action de moyens (12) exerçant sur lui une force tendant à l'entraîner vers la seconde position, par des moyens de retenue (6) susceptibles d'être commandés de l'extérieur pour libérer le mouvement dudit piston sous l'action des premiers moyens susmentionnés,

— l'aiguille (10) étant entièrement escamotée à l'intérieur de la partie antérieure dudit corps de piston (4) dans ladite première position,

— la course du piston étant telle que, lorsque la partie antérieure du dispositif est placée et maintenue par le manipulateur, directement ou par le truchement d'un système externe de support co-agissant avec ce dispositif contre le corps du malade ou à une distance déterminée du corps du malade, au niveau de l'os qui doit être traversé par l'aiguille, l'extrémité de l'aiguille soit apte à sortir du corps de piston au niveau de son extrémité antérieure (32), notamment à travers un opercule (14) ou analogue, à traverser l'épaisseur de l'os et à atteindre la région de la moelle où devra s'effectuer le prélèvement, lorsque le piston aura été soustrait à l'action desdits moyens de retenue (6) par l'intermédiaire des moyens (22, 28) commandés de l'extérieur et caractérisé

. en ce que l'aiguille (10) est une aiguille de prélèvement traversant le piston (2)

. en ce que la partie postérieure du corps de piston, du côté du piston opposé à celui qui est traversé par l'aiguille; définit une chambre fermée (16), permettant alors la réception de la moelle aspirée dans cette chambre par l'intermédiaire de l'aiguille de prélèvement, sous l'effet de la dépression qui est alors formée consécutivement au déplacement du piston de sa première position vers sa deuxième position.

2. Dispositif selon la revendication 1, caractérisé en ce que les moyens (12) exerçant sur le piston (2) une force tendant à l'entraîner vers la susdite seconde position, sont du type élastique.

3. Dispositif selon la revendication 2 caractériséen ce que les susdits moyens sont constitués par un ressort (12) travaillant en traction et exerçant ses effets directement sur le piston (2).

4. Dispositif selon l'une quelconque des revendications 1 à 3, caractérisé en ce que les moyens (6) susceptibles d'être commandés de l'extérieur et qui assurent la retenue du piston dans sa première position, sont constitués par des doigts ou ergots escamotables vers la surface intérieure du corps de piston.

5. Dispositif selon l'une quelconque des revendications 1 à 4, caractérisé par le fait qu'il comporte au moins un compartiment distinct (18) susceptible d'être mis en communication avec la susdite chambre (16), ce compartiment distinct pouvant contenir des réactifs solides ou liquides.

6. Dispositif selon la revendication 5, caractérisé en ce que le compartiment distinct (18) est séparé de ladite chambre (16) par une membrane ou pellicule (20), fracturable sous l'effet de la dépression produite dans ladite chambre, sous l'effet du déplacement du piston (2) de la première à la seconde position susdite.

7. Ensemble comprenant le dispositif selon l'une quelconque des revendications 1 à 6, sous la forme d'une cartouche (4) jetable et remplaçable, et d'un système de support (23) externe pour ladite cartouche, comprenant un logement (21) dans lequel celle-ci peut être introduite et duquel elle peut ensuite être retirée, ledit système de support étant pourvu desdits moyens de commande (28), notamment par gâchette, susceptibles d'être mis en liaison avec les moyens de retenue (6) du piston (2) à l'occasion de l'introduction de ladite cartouche (4) dans le susdit logement (21), pour être ensuite apte à commander, notamment par actionnement de la gâchette (28), la libération du mouvement du piston (2), et une poignée convenablement placée de façon à permettre l'application de la partie antérieure de la cartouche ou de rebords correspondants dudit système qui lui sont associés, de manière à assurer un contact correct et adhérent à la peau du malade.

## Patentansprüche

1. Knochenmark-Punktiervorrichtung mit einer Nadel (10), die mit einem Kolben (2) fest verbunden ist, der sich zwischen einer ersten Lage und einer zweiten Lage im Inneren eines einem vorderen Abschnitt vorgesehenen Kolbenkörpers oder Rohrs (4) verschieben kann, wobei

— der Kolben (2) gegen die Wirkung einer Einrichtung (12) die auf ihn eine Kraft ausübt, die die Tendenz hat, ihn in die zweite Lage zu treiben, durch eine Halteeinrichtung (6) in seiner ersten Lage gehalten wird, die von außen betätigt werden kann, um die Bewegung des Kolbens unter Wirkung der oben erwähnten ersten Einrichtung freizugeben,

— die Nadel (10) in der ersten Lage vollständig in das Innere des vorderen Abschnitts des Kolbenkörpers (4) eingezogen ist,

— der Hub des Kolbens so ausgelegt ist, daß, wenn der vordere Abschnitt der Vorrichtung von einer Bedienungsperson direkt oder mittels eines äußeren, mit der Vorrichtung zusammenwirkenden Halterungssystems auf Höhe des Knochens, durch den die Nadel hindurchgehen soll, gegen den Körper eines Patienten oder in einem vorbestimmten Abstand zum Körper des Patienten angeordnet und gehalten wird, die Spitze der Nadel aus dem Kolbenkörper auf Höhe seines vorderen Endes (32), insbesondere durch einen Deckel (14) oder dergleichen, austreten kann, durch die Dicke des Knochens hindurchgehen und den Bereich des Knochenmarks erreichen kann, wo die Entnahme erfolgen soll, wenn der Kolben der Wirkung der Halteeinrichtung mittels einer von außen betätigten Einrichtung (22, 28) entzogen worden ist, dadurch gekennzeichnet,

— daß die Nadel (10) eine durch den Kolben (2) hindurchgehende Entnahme-Nadel ist,

— daß der hintere Abschnitt des Kolbenkörpers auf der Seite des Kolbens, die der gegenüberliegt, durch die die Nadel hindurchgeht, eine abgeschlossene Kammer (16) bildet, wodurch die Aufnahme von abgesaugtem Knochenmark in diese Kammer über die Entnahme-Nadel unter Wirkung des Unterdrucks gestattet wird, der als Folge der Verschiebung des Kolbens von seiner ersten Lage in Richtung seiner zweiten Lage gebildet wird.

2. Vorrichtung nach Anspruch 1, dadurch gekennzeichnet, daß die Einrichtung (12), die auf den Kolben (2) eine Kraft ausübt, die die Tendenz hat, ihn in die zweite Lage zu treiben, elastischer Art ist.

3. Vorrichtung nach Anspruch 2, dadurch gekennzeichnet, daß die Einrichtung von einer Feder (12) gebildet wird, die unter Zug arbeitet, und ihre Wirkungen direkt auf den Kolben (2) ausübt.

4. Vorrichtung nach einem der Ansprüche 1 bis 3, dadurch gekennzeichnet, daß die Einrichtung (6), die von außen betätigt werden kann, und die das Halten des Kolbens in seiner ersten Lage sichert, von Zapfen oder Vorsprüngen gebildet wird, die in Richtung der Innenfläche des Kolbenkörpers einziehbar sind.

5. Vorrichtung nach einem der Ansprüche 1 bis 4, gekennzeichnet durch wenigstens einen abgetrennten Raum (18), der mit der Kammer (16) in Verbindung setzbar ist, wobei der abgetrennte Raum feste oder flüssige Reagenzien enthalten kann.

6. Vorrichtung nach Anspruch 5, dadurch gekennzeichnet, daß der abgetrennte Raum (18) von der Kammer (16) durch eine Membrane oder eine Folie (20) getrennt ist, die unter Wirkung des in der Kammer erzeugten Unterdrucks aufgrund der Verschiebung des Kolbens (2) von der ersten Lage in die zweite Lage brechen kann.

7. Anordnung mit der Vorrichtung nach einem der Ansprüche 1 bis 6, in Form einer wegwerfbaren und austauschbaren Hülse (4) und eines äußeren Halterungssystems (23) für die Hülse, das eine Aufnahme (21), in die die Hülse eingesetzt und aus der sie danach wieder herausgezogen werden kann, wobei das Halterungssystem mit einer insbesondere durch einen Drücker betätigbaren Betätigungseinrichtung (28) versehen ist, die im Falle der Einführung der Hülse (4) in die Aufnahme (21) mit der Halteeinrichtung (6) des Kolbens (2) in Verbindung gebracht werden kann, um anschließend, insbesondere durch die Aktivierung des Drückers (28), die Freigabe der Kolbenbewegung (2) zu veranlassen, und einen Handgriff aufweist, der zweckmäßigerweise so angebracht ist, daß er die Anbringung des vorderen Abschnitts der Hülse oder entsprechender Ränder des Systems erlaubt, die ihm so zugeordnet sind, daß sie einen einwandfreien und haftenden Kontakt auf der Haut des Patienten sichern.

## Claims

1. Apparatus for bone marrow puncture comprising a needle (10) fused to a piston (2), the piston being capable of being displaced between a first and a second position in the interior of the barrel of the piston or tube (4), equipped with an anterior part,

— this piston (2) being maintained in its first position against the action of means (12) exerting on it a force tending to drive it towards the second position by restraining means (6) liable to be externally controlled in order to release the movement of said piston under the action of the first means abovementioned,

— the needle (10) being entirely retracted within the interior of the anterior part of the said barrel of the piston (4) in the said first position,

— the stroke of the piston being such that, when the anterior part of the instrument is placed and maintained by the operator directly or with the aid of an external system of support harnessed to this instrument against the body of the patient or at a specified distance from it, at the height of the bone which has to be pierced by the needle, the extremity of the needle should be capable of projecting from the body of the piston at its anterior end (32), in particular through a percussion cap (14) or something similar, passing through the thickness of the bone and reaching the area of the bone marrow where the sample is to be taken, when the piston will have been released from the braking mechanism (6) by the intermediary of means (22, 28) externally controlled, and characterized

. in that the needle (10) is a sampling needle traversing the piston (2),

. in that the posterior part of the piston barrel, on the opposite side of the piston to that to which the needle is joined, defines a closed chamber (16), then allowing the aspirated bone marrow to be collected through the intermediary of the sampling needle, as a result of the effect of the depression subsequently generated by the displacement of the piston from the first to the second position.

2. Apparatus according to claim 1, characterized in that the means (12) exerting a force on the piston (2) tending to drive it to the above-mentioned second position are of an elastic design.

3. Apparatus according to claim 2, characterized in that the above-mentioned means consist of a spring (12) working in traction and exerting its effect directly on the piston (2).

4. Apparatus according to any one of the claims 1 to 3, characterized in that the means (6) which can be controlled from the exterior and which maintained the piston in the first position consist of stopping pins or catches which are retractable towards the internal surface of the piston barrel.

5. Apparatus according to any one of the claims 1 to 4, characterized by the fact that it comprises at least on distinct compartment (18) which can be connected with the above-mentioned chamber (16); this distinct compartment may contain solid or liquid reagents.

6. Apparatus according to claim 5, characterized in that the distinct compartment (18) is separated from the said chamber (16) by a membrane or film (20) which can be broken as a consequence of the depression generated in the said chamber by the displacement of the piston (2) from the first to the second position mentioned above.

7. Assembly comprising the apparatus according to any one of the claims 1 to 6, in the form of a disposable and replaceable cartridge (4) and an external system of support (23) for the said cartridge, comprising a housing (21) into which the cartridge may be introduced and from which it may be withdrawn, said support system being equipped with said control means (28), in particular a trigger which can be connected to the restraining means (6) of the piston (2) when said cartridge (4) is introduced into the above-mentioned housing (21) in order to be able to control, in particular by releasing the trigger (28), the displacement of the piston (2), and a handle suitably placed so as to allow the anterior part of the cartridge or the corresponding edges of the said support system which is combined with it, to be applied in a manner which ensures correct and direct contact with the skin of the patient.

FIG.1.

FIG.2.

FIG.3.